(19) Europäisches Patentamt

European Patent Office

Office européen des brevets

(11) **EP 1 093 581 B1**

(12) **FASCICULE DE BREVET EUROPEEN**

(45) Date de publication et mention de la délivrance du brevet:
**07.07.2004 Bulletin 2004/28**

(51) Int Cl.⁷: **G01N 33/12**, G01N 21/31, G01N 21/35

(21) Numéro de dépôt: **99925076.4**

(22) Date de dépôt: **14.06.1999**

(86) Numéro de dépôt international:
**PCT/FR1999/001404**

(87) Numéro de publication internationale:
**WO 2000/002043 (13.01.2000 Gazette 2000/02)**

(54) **PROCEDE ET DISPOSITIF DE PREDICTION DE LA TENDRETE D'UNE VIANDE**

VERFAHREN UND VORRICHTUNG ZUM VORHERSAGEN DER ZARTHEIT VON FLEISCH

METHOD AND DEVICE FOR DETERMINING MEAT TENDERNESS

(84) Etats contractants désignés:
**AT BE CH CY DE DK ES FI FR GB GR IE IT LI LU MC NL PT SE**

(30) Priorité: **03.07.1998 FR 9808536**

(43) Date de publication de la demande:
**25.04.2001 Bulletin 2001/17**

(73) Titulaire: **SOCIETE VITREENNE D'ABATTAGE F-35500 Vitre (FR)**

(72) Inventeurs:
  • **AIGNEL, Denis**
    **F-35510 Cesson-Sévigné (FR)**
  • **FAURE, Patrick**
    **F-35760 Saint-Grégoire (FR)**
  • **LAUMONIER, Patrice**
    **F-35500 Balaze (FR)**

(74) Mandataire: **Plaçais, Jean-Yves et al**
    **Cabinet Netter,**
    **36, avenue Hoche**
    **75008 Paris (FR)**

(56) Documents cités:
    **EP-A- 0 402 877        EP-A- 0 444 675**
    **WO-A-94/00997        WO-A-98/20339**

    • **NAES T ET AL: "COMPARISON OF MULTIVARIATE CALIBRATION AND DISCRIMINANT ANALYSIS INEVALUATING NIR SPECTROSCOPY FOR DETERMINATION OF MEAT TENDERNESS" APPLIED SPECTROSCOPY, vol. 51, no. 3, mars 1997 (1997-03), pages 350-357, XP000698671**
    • **CHEN Y -R ET AL: "TRANSPORTABLE SPECTROPHOTOMETER SYSTEM FOR ON-LINE CLASSIFICATION OF POULTRY CARCASSES" APPLIED SPECTROSCOPY, vol. 50, no. 7, juillet 1996 (1996-07), pages 910-916, XP000642347**

EP 1 093 581 B1

## Description

La présente invention concerne la prédiction de la tendreté d'une viande, en particulier de type bovin, sur le site de transformation, à l'aide d'informations biologiques et/ou physico-chimiques et de mesures optiques dans le domaine du visible et du proche infrarouge.

Parmi les nombreux facteurs de qualité d'une viande, telle que la jutosité, la tendreté, la couleur, ou la flaveur, la tendreté est considérée comme étant le facteur de qualité qui procure le plus de satisfaction au consommateur.

Il est donc économiquement important pour les industriels de la viande de pouvoir prédire la tendreté d'une viande, rapidement et le plus tôt possible dans le processus de transformation de la viande en milieu industriel (abattoir). De plus et surtout, cette prédiction doit être fiable afin de la garantir jusqu'au stade de consommation.

On connaît déjà de nombreux procédés industriels de prédiction de la qualité d'une viande, notamment de type non destructif (c'est-à-dire sans découpe d'échantillons).

Dans le Brevet FR 1 550 169, une sonde détermine les couleurs d'une viande au moyen d'une lumière transmise par une pièce de viande soumise à observation. L'instrument comprend une branche d'émission et une branche de réception destinées à être introduites dans la viande. Une lampe délivre une lumière par la branche d'émission située en regard de la branche de réception. Un tel document ne précise pas les longueurs d'ondes de la lumière utilisée. De plus, seules les couleurs de la viande sont déterminées, ce qui est insuffisant pour prédire la tendreté de la viande avec un degré de fiabilité satisfaisant. insuffisant pour prédire la tendreté de la viande avec un degré de fiabilité satisfaisant.

Dans la publication EP-A-0402877, une sonde est introduite dans un morceau de viande pour déterminer sa qualité. La technique utilisée est la mesure de l'intensité lumineuse réfléchie (réflectance) en lumière visible ou proche infra-rouge. Une telle sonde permet seulement d'estimer les concentrations d'un ou plusieurs composants de la viande, ce qui est insuffisant pour prédire la tendreté de ladite viande avec un degré de fiabilité satisfaisant.

La présente invention a pour but d'améliorer la fiabilité des techniques antérieures de prédiction de la qualité et plus précisément de déterminer la tendreté sur le site de transformation.

Elle porte sur un procédé de prédiction de la qualité d'une viande, en particulier de type bovin, pouvant être utilisée en ligne sur le site de transformation.

Selon une définition générale de l'invention, le procédé comprend les étapes suivantes :

- a) recueillir, sur le site de transformation, des données relatives à des paramètres appartenant au groupe formé par la race, l'âge, et la catégorie de l'animal, ainsi qu'à des paramètres biologiques et/ou physico-chimiques de la carcasse de l'animal appartenant au groupe formé par le poids, la conformation, l'état d'engraissement, le pH et la couleur de la carcasse, ainsi que l'épaisseur du cuir,

- b) obtenir au moins un spectre optique de la viande correspondant à des longueurs d'onde appartenant à une plage spectrale allant du visible jusqu'au proche infrarouge, et

- c) combiner les données obtenues lors des étapes a) et b), en vue de prédire la tendreté de la viande selon une loi de prédiction prédéterminée établie par rapport à une échelle de référence de tendreté prédéterminée.

La Demanderesse a observé que certains paramètres de l'animal et de sa carcasse, tels que la race, l'âge, et la catégorie de l'animal, ainsi que le poids, la conformation, l'état d'engraissement, le pH, la couleur de la carcasse, et l'épaisseur du cuir, etc, sont des données qui sont corrélées à la tendreté.

Or, jusqu'à présent aucun modèle de prédiction ne les utilise directement, probablement en raison de la méconnaissance ou du désintérêt à leur égard chez les scientifiques.

En dépit de ce préjugé, la Demanderesse les utilise pour les combiner en outre avec des mesures optiques en vue de prédire avec une meilleure fiabilité la tendreté d'une viande sur le site de transformation.

Les données (race, âge, catégorie de l'animal, poids, conformation, état d'engraissement, pH, couleur de la carcasse, épaisseur du cuir, etc), sont appelées ci après "données Expert", parce qu'elles proviennent d'une connaissance approfondie de l'animal et des caractéristiques qui influencent la tendreté.

Le procédé selon l'invention permet de fournir une prédiction objective et non destructive de la tendreté d'une viande, pouvant être mise en place en ligne sur le site de transformation en milieu industriel sans faire appel à des dispositifs sophistiqués et coûteux.

De plus, les résultats de la prédiction selon l'invention sont suffisamment fiables pour permettre de garantir la tendreté avec une forte probabilité en sortie du site de transformation de la viande en milieu industriel.

En pratique, l'étape b) est réalisée en mode réflexion et/ou en mode transmission.

En mode transmission, l'étape b) comprend les étapes suivantes :

- b1) prévoir une sonde comprenant une branche d'émission et une branche de réception, espacées l'une de l'autre d'une distance prédéterminée ;

- b2) insérer les branches d'émission et de réception dans un morceau de viande choisi, à une profondeur choisie;

- b3) éclairer le morceau de viande à l'aide de la branche d'émission ainsi insérée dans le morceau de viande, selon un rayonnement lumineux large bande en fréquence allant du visible jusqu'au proche infrarouge;

- b4) recevoir la lumière transmise par le morceau de viande à l'aide de la branche réceptrice ainsi insérée dans le morceau de viande ; et

- b5) enregistrer un spectre de transmission du morceau de viande allant du visible jusqu'au moins le proche infrarouge.

En mode réflexion, la sonde comprend une seule branche assurant l'émission et la réception de la lumière incidente et de la lumière réfléchie respectivement.

En pratique, la loi de prédiction est établie sur une série significative de différents morceaux de viande de différents animaux pour chacun desquels des données spectrales et non spectrales sont obtenues selon les étapes a) et b) et comparées à une échelle de référence établie au moyen d'informations sensorielles et/ou de force de cisaillement et/ou de force de compression mesurées sur ladite série significative de différents morceaux de viande.

De préférence, la prédiction est réalisée selon une méthode statistique multidimensionnelle telle que la méthode des moindres carrés partiels, en vue d'obtenir un modèle mathématique de prédiction destiné à être utilisé sur chaque morceau de viande dont la tendreté est à prédire.

Selon une autre caractéristique du procédé selon l'invention, l'étape b) est réalisée sur carcasse, quartiers, muscles, ou steaks.

La présente invention a également pour objet un dispositif de prédiction de la qualité d'une viande, en particulier de type bovin, sur le site de transformation.

Selon une définition générale du dispositif selon l'invention, le dispositif comprend :

- des moyens pour recueillir, sur le site de transformation, des données relatives à des paramètres appartenant au groupe formé par la race, l'âge, et la catégorie de l'animal, ainsi qu'à des paramètres biologiques et/ou physico-chimiques de la carcasse de l'animal appartenant au groupe formé par le poids, la conformation, l'état d'engraissement, le pH et la couleur de la carcasse, ainsi que l'épaisseur du cuir,

- des moyens pour obtenir au moins un spectre optique de la viande à des longueurs d'onde appartenant à une plage spectrale allant du visible jusqu'au proche infra-rouge, et

- des moyens de traitement pour combiner les données obtenues lors des étapes a) et b) en vue de prédire la tendreté de la viande selon une loi de prédiction prédéterminée établie par rapport à une échelle de référence de tendreté prédéterminée.

D'autres caractéristiques et avantages de l'invention apparaîtront à la lumière de la description détaillée ci-après et des dessins dans lesquels :

- la figure 1 illustre schématiquement une ligne en continu d'un abattoir industriel sur laquelle est mise en place l'invention;

- la figure 2 représente des spectres d'absorbance en réflexion obtenus sur des vaches de tendreté différente selon l'invention;

- la figure 3 illustre schématiquement les moyens essentiels et constitutifs d'un dispositif d'obtention d'un spectre d'une viande en transmission selon l'invention;

- la figure 4 est une vue en coupe illustrant une sonde en transmission selon l'invention; et

- la figure 5 est une vue en coupe illustrant les moyens constitutifs d'une sonde en réflexion selon l'invention.

Les dessins comportent pour l'essentiel des éléments de caractère certain. A ce titre, ils pourront non seulement servir à mieux faire comprendre la description détaillée ci-après, mais aussi contribuer, le cas échéant, à la définition de l'invention.

La présente invention est destinée plus particulièrement à être mise en oeuvre sur un site de transformation de viande (abattage et découpe), par exemple sur la ligne en continu d'un abattoir industriel.

Elle s'applique à tout type de viande, notamment de type bovin, ovin, caprin, porcin, ou analogue.

D'une manière générale, un site d'abattage et de découpe, comprend un poste de réception des animaux vivants, un poste d'abattage, des postes de préparation des animaux abattus en carcasse, des postes de découpe au moins en demi-carcasse, une phase de ressuage, et le cas échéant des postes de découpe et de préparation en quartiers, muscles, ou steaks.

Certaines caractéristiques ou paramètres de l'animal et de sa carcasse, sont connus ou facilement mesurables sur le site de transformation.

Par exemple, la race RA (charolais, limousine, ...), l'âge AG, la catégorie CA (jeune bovin, boeuf, vache, génisse,...) de l'animal sont recueillis au niveau du poste de réception de l'animal vivant. Ces informations, ainsi que l'origine de l'animal NE, sont disponibles sur la carte d'identité de l'animal.

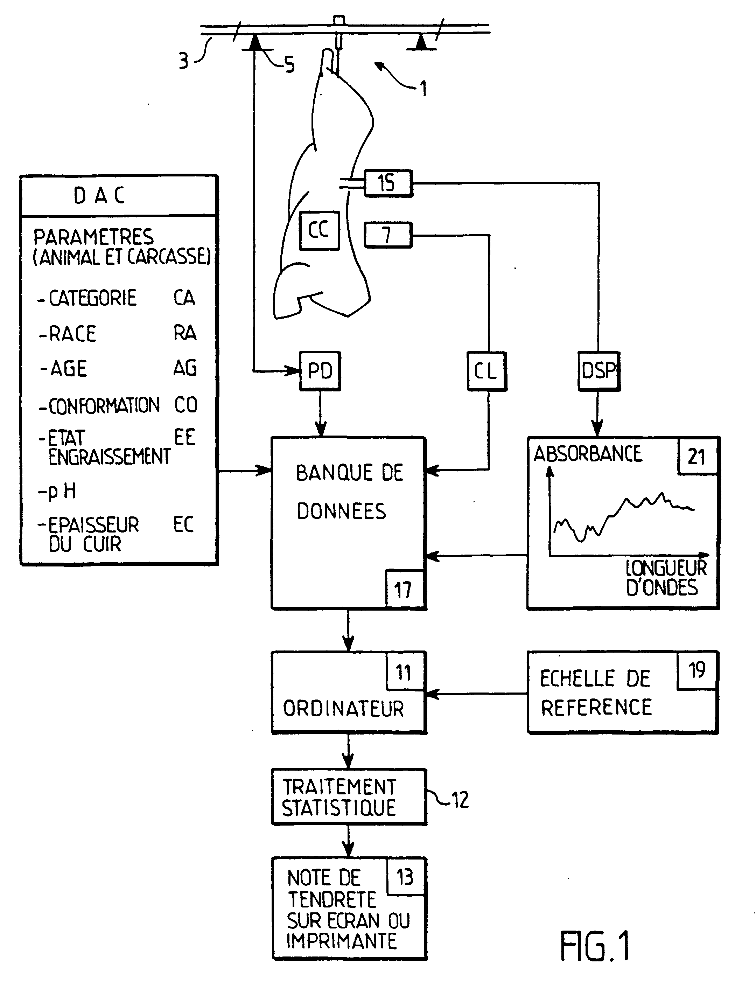

En référence à la figure 1, des caractéristiques ou paramètres biologiques et/ou physico-chimiques de la carcasse de l'animal sont mesurés sur le site de transformation, notamment sur la ligne en continu 1 d'un abattoir industriel.

Parmi ces caractéristiques, on trouve le poids PD de la carcasse qui est mesuré à l'aide d'un dispositif de pesée 5 placé sur le convoyeur aérien 3 de la ligne en continu 1, par exemple avant le poste de ressuage.

La conformation CO de la carcasse CC est également évaluée. 5 niveaux de conformation CO sont prévus selon la grille de classification EUROP, dont les critères sont notamment décrits dans le règlement N°1208/81 du Conseil des Communautés Européennes du 28 avril 1981 établissant la grille communautaire de classement des carcasses des gros bovins ainsi que dans le règlement N°1026/91 du Conseil des Communautés Européennes du 22 avril 1991.

L'état d'engraissement EE de la carcasse CC est également évalué. 5 niveaux varient entre "très faible" et "très bon". En pratique, le niveau d'engraissement EE est évalué avant le poste de ressuage selon les critères notamment décrits dans le règlement N°1208/81 du Conseil des Communautés Européennes du 28 avril 1981 mentionné ci-avant.

La couleur CL de la carcasse CC est également mesurée, par exemple avant le poste de ressuage. La mesure de la couleur CL peut être obtenue par un colorimètre 7. Un expert peut également, à l'oeil nu, mesurer la couleur CL de la carcasse CC.

Le pH (degré d'acidité) de la carcasse CC peut être également mesuré à l'aide d'un instrument de mesure d'acidité (non représenté), à différents instants après abattage, par exemple à 1, 2, 3, 8, 12, ou 24 heures après abattage. Par exemple, le pH de la carcasse est mesuré selon la norme française NF V 46-001 de décembre 1996, intitulée "Viandes de gros bovins - conditions de valorisation du potentiel de tendreté".

Une autre donnée intéressante pour la prédiction de la tendreté, comme on le verra plus en détail ci-après, est l'épaisseur du cuir EC qui peut être relevée après abattage au moyen de la pesée du cuir combinée ou non avec une mesure de la surface de celui-ci.

Toutes ces données DAC, appelées données Expert, sont enregistrées et stockées dans une mémoire d'un ordinateur 11 relié à une imprimante et un écran 13.

La Demanderesse a observé qu'il peut être avantageux d'intégrer ces données Expert DAC dans un modèle de prédiction de la tendreté.

De plus, les résultats de la prédiction sont encore améliorés lorsque ces données expert DAC sont combinées avec des données optiques spectrales DSP relatives à la réflexion et/ou transmission de la viande dans le domaine du visible et du proche infrarouge.

Il est à remarquer qu'en présence d'animaux ayant sensiblement les mêmes caractéristiques biologiques et/ou physico-chimiques DAC, la prédiction de la tendreté peut être réalisée, avec un degré de fiabilité relativement satisfaisant, sur la seule base de mesures optiques allant du visible jusqu'au proche infrarouge.

Par ailleurs, en présence d'animaux de caractéristiques biologiques et/ou physico-chimiques hétérogènes, le nombre de caractéristiques biologiques et/ou physico-chimiques à combiner dépend du degré de fiabilité de la prédiction de la tendreté à atteindre.

Ainsi, le degré de fiabilité de la prédiction qui intégré de nombreux paramètres DAC sera meilleur que celui d'une prédiction qui intégré peu de paramètres DAC.

Les mesures spectrales DSP peuvent être obtenues sur le site de transformation, avant, pendant ou après le ressuage, et sur carcasse, quartiers, muscles ou steaks, au moyen d'un appareil 15 approprié.

L'ordinateur 11 est apte à prédire la tendreté du morceau de viande ainsi observé selon une loi de prédiction de tendreté prédéterminée, en vue d'afficher ou imprimer le niveau de tendreté du morceau de viande sur un périphérique 13 approprié.

En pratique, la loi de prédiction est établie sur une série significative de différents morceaux de viande (faux filet, rond de gîte, etc) de différents animaux (vaches, jeunes bovins, etc) pour chacun desquels des données spectrales DSP et non spectrales DAC sont obtenues et comparées à une échelle de référence choisie 19.

Les données spectrales DSP sont obtenues au moyen d'au moins une mesure optique de la lumière transmise et/ou réfléchie par le morceau de viande en mode transmission et/ou réflexion dont on décrira plus en détail un exemple. La mesure optique est avantageusement réalisée dans la gamme 400-2500 nm, le cas échéant limitée à 400-2100 nm.

Les données spectrales DSP de différents morceaux de viande de différents animaux sont stockées dans une mémoire 21 en association avec les données non spectrales DAC de chaque animal. Toutes ces données DAC et DSP sont gérées dans une base de données 17.

Pour chaque vache (ou autre animal), on dispose des données DAC issues de la base de données 17. Les données spectrales DSP et non spectrales DAC ainsi obtenues sont comparées à une échelle de référence choisie 19.

Par exemple, l'échelle de référence 19 est établie à l'aide d'informations sensorielles et/ou de force de cisaillement et/ou de force de compression obtenues sur chaque morceau de viande de chaque animal participant à l'établissement de la loi de prédiction.

Les informations de tendreté de référence 19 sont stockées dans la base de données 17 reliée à l'ordinateur 11.

Les forces de cisaillement sont établies par exemple selon une méthode de type WARNER BRATZLER, définie par WHEELER, KOOHMARAIE, CUNDIF et DIKE en 1994 dans leur étude intitulée "Effects of cooking and shearing methodology on variation in Warner Bratzler shear force values in beef" parue dans la revue "Journal of Animal Science", N°72, 1994.

En variante, la force de compression est établie sur des lamelles de steaks bouillis selon la méthode VOLODKEWICH.

L'échelle de référence 19 peut être évaluée aussi à l'aide d'un jury de dégustation, comme décrit dans "Research Guidelines for Cookery, Sensory Evaluation and Instrumental Tenderness Measurements of Fresh Meat", publiée par l'American Meat Science Association, édition 1995.

Dans le cas de faux filet de vache, la tendreté de chacun d'eux est évaluée par exemple par une mesure de la force de cisaillement selon la méthode Warner Bratzler mentionnée ci-avant.

Une analyse est ensuite conduite sur chaque spectre optique selon une méthode de traitement statistique choisie 12, par exemple une "Analyse en Composantes Principales dite ACP". Cette analyse ACP permet d'isoler par exemple les 10 premières composantes principales qui vont constituer les variables spectrales DSP du modèle spectral selon l'invention.

En pratique, chaque paramètre (données spectrales DSP ou Expert DAC) est considérée comme une variable individuelle utilisée dans un modèle mathématique 12 (par exemple régression multiple) pour prédire la tendreté.

La loi ou modèle de prédiction peut être du type :

$$\text{Prédiction} = C + a_1\, X_1 + a_2\, X_2 + ... + a_n\, X_n$$

où C est une constante,
$a_1$ à $a_n$ sont des coefficients, et $X_1$ à $X_n$ sont les variables spectrales DSP et non spectrales DAC.

Une expérimentation a été conduite sur 100 faux filets de vaches. Le modèle mathématique prenant en compte l'ensemble du spectre de 400 à 2500 nm en mode réflexion et les données DAC permet d'obtenir une prédiction de la tendreté avec un coefficient de corrélation multiple $R^2$ de 0.62.

Des modèles prédictifs plus fiables ont été établis sur des sous populations de cet échantillon. Par exemple, les 25 vaches de race charolaise, limousine ou croisée, la prédiction de la tendreté est associée à un coefficient de corrélation $R^2$ de 0.95. Ce coefficient fait intervenir par ordre d'importance pour les variables non spectrales DAC, les paramètres suivants :

- couleur du faux filet CL,
- épaisseur du cuir EC,
- poids de la carcasse PD,
- conformation CO,
- pH au cours du ressuage,
- âge de l'animal AG.

Pour les données spectrales, les composantes principales 3, 7, 11, 5, 10, et 2 sont les plus significatives. Elles comprennent les longueurs d'ondes généralement utilisées pour la prédiction des protéines, de la matière grasse, de la teneur en eau, du collagène ainsi que du pigment de la viande. Par exemple, il s'agit des longueurs d'ondes suivantes : 460, 660, 800, 880, 980, 1020, 1120, 1320, 1580, 1920 nm.

Chacun de ces paramètres est corrélé de façon significative à la tendreté. Toutefois, à eux seuls ils ne permettent pas de garantir la tendreté avec une forte probabilité. Ainsi, par exemple, la couleur du faux filet est corrélée à la tendreté avec un coefficient de corrélation $R^2$ de 0.65 tandis que l'épaisseur du cuir est corrélée à la tendreté avec un coefficient de 0.43.

Dans un autre exemple de réalisation de l'invention, la Demanderesse a démontré que les données spectrales DSP en mode transmission permettent d'obtenir une bonne prédiction de la tendreté ($R^2$ de l'ordre de 0.6), sur des ronds de gîte de 100 jeunes bovins. Ce coefficient est amélioré en intégrant certaines données expert DAC. Par exemple, un coefficient de corrélation de 0.95 a été obtenu pour des ronds de gîte (semitendinosus) de 24 jeunes bovins de race laitière.

Ce coefficient fait intervenir par ordre d'importance pour les variables non spectrales, les paramètres suivants :

- pH au cours du ressuage,
- couleur de la carcasse CL,
- état d'engraissement EE,
- épaisseur du cuir EC,
- poids de la carcasse PD,
- âge de l'animal AG.

Dans cet exemple en mode transmission, certaines longueurs d'ondes prépondérantes correspondent aux longueurs d'ondes généralement utilisées pour la prédiction du pigment de la viande (560 nm), de la matière grasse (930 et 1215 nm) et la teneur en eau (1682 nm).

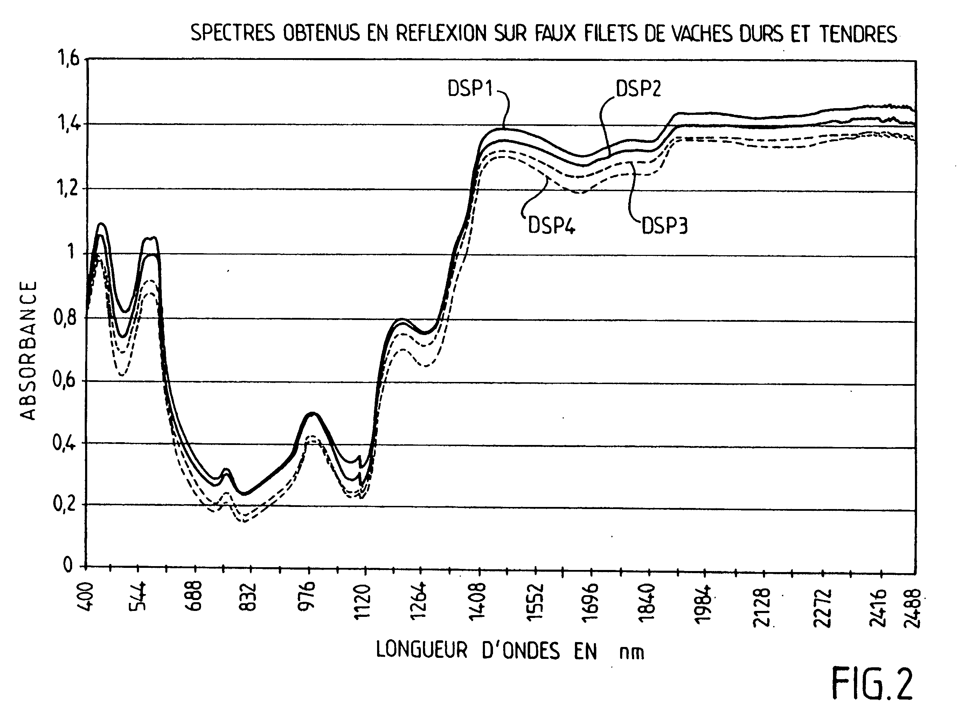

En référence à la figure 2, on a représenté différents spectres d'absorbance de faux filet de vaches dures et tendres du visible (400 nm) au proche infrarouge (2480 nm), en mode réflexion.

On observe que certains pics d'absorbance sont fortement corrélés à la tendreté. Il est à noter qu'il est souhaitable sur le plan de la fiabilité de mesurer un spectre optique complet au moins de 400 à 2100 nm.

Le spectre DSP1 est celui d'un faux filet d'une vache dont la tendreté est évaluée à 6,10 sur l'échelle de référence de type WARNER BRATZLER, c'est à dire un faux filet dur.

Le spectre DSP2 est celui d'un faux filet d'une vache dont la tendreté est évaluée à 6,90 sur l'échelle de référence de type WARNER BRATZLER, c'est à dire un faux filet dur.

Le spectre DSP3 est celui d'un faux filet d'une vache dont la tendreté est évaluée à 2,9 sur l'échelle de référence de type WARNER BRATZLER, c'est à dire un faux filet tendre.

Le spectre DSP4 est celui d'un faux filet d'une vache dont la tendreté est évaluée à 3,30 sur l'échelle de référence de type WARNER BRATZLER, c'est à dire un faux filet tendre.

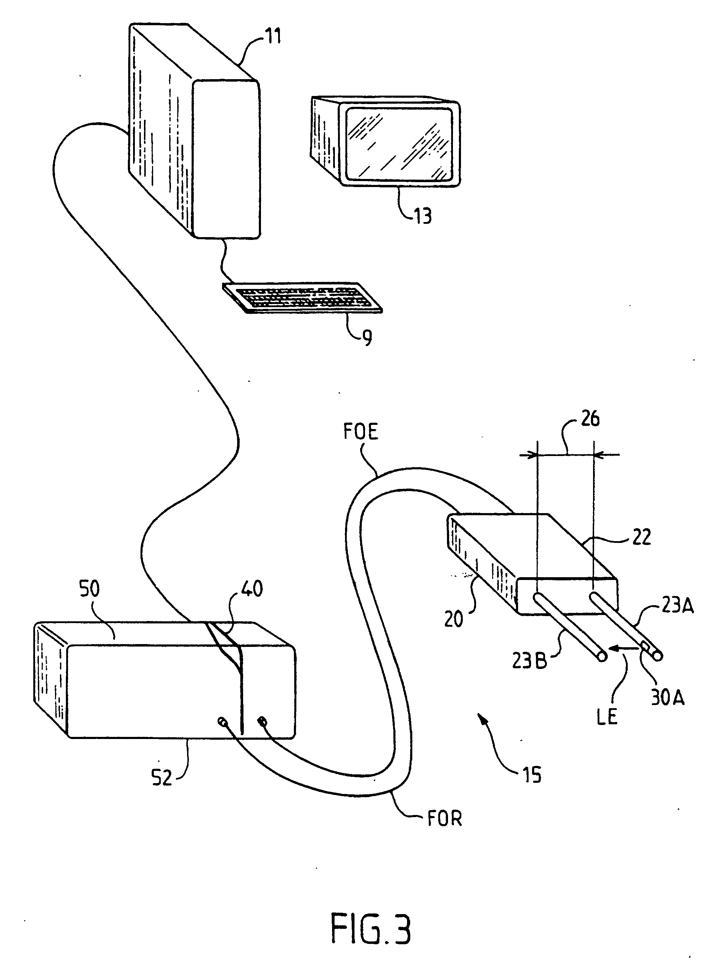

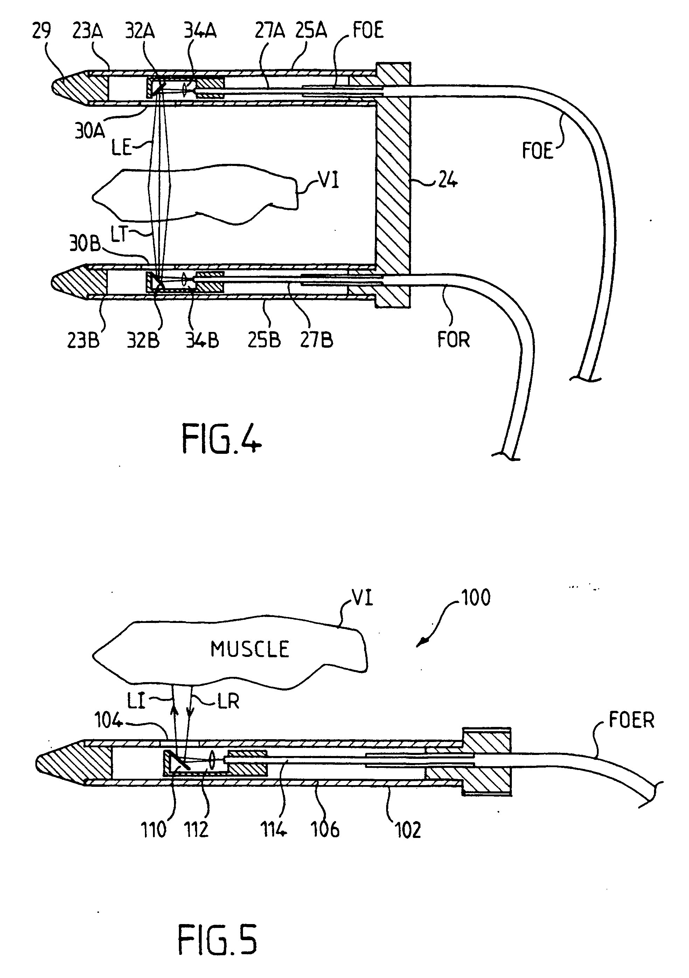

En référence aux figures 3 et 4, on a décrit un dispositif d'obtention de données spectrales, fonctionnant en mode de transmission.

Le dispositif d'obtention de données spectrales 15 comprend par exemple un instrument de type pistolet-piqueur 20 comportant un boîtier 22 et une sonde 23 à deux branches 23A et 23B, l'une 23A étant dédiée à l'émission et l'autre 23B étant dédiée à la réception. Les branches 23A et 23B de la sonde sont destinées à être insérées dans un morceau de viande choisi, à une profondeur choisie. Les branches 23A et 23B sont parallèles entre elles et espacées l'une de l'autre d'une distance prédéterminée. Les deux branches sont reliées l'une à l'autre par une base 24 formant soutien et solidaire du boîtier 22. La base 24 joue aussi le rôle d'interface pour les fibres optiques que l'on décrira plus en détail ci-après.

Avantageusement, l'entre-axe 26 entre les deux branches 23A et 23B est réglable, entre une distance minimum de l'ordre de 10 mm et une distance maximale de 40 mm.

Il est à remarquer que l'entre-axe 26 entre les deux branches est réglable mais toujours fixé lors de la mesure. La profondeur de pénétration de la sonde peut être ajustée entre 5 et 15 cm. Le diamètre des branches pénétrantes est de l'ordre de 1 cm environ.

Avantageusement, les extrémités pénétrantes des branches sont équipées de bouchons 29 en acier inoxydable.

Les longueurs des branches 23A et 23B sont de l'ordre de 20cm.

La branche 23A comprend une fenêtre 30A disposée sur le côté interne de son extrémité pénétrante, perpendiculairement à l'axe de pénétration tandis que la branche 23B comprend une fenêtre 30B disposée sur le côté interne de son extrémité pénétrante, perpendiculairement à l'axe de pénétration, et en regard de la fenêtre 30A.

Chaque branche comprend un tube, référencé 25A pour la branche 23A, et 25B pour la branche 23B. Les tubes sont creux, par exemple en silice.

Le rayonnement lumineux LE est émis par une source de lumière distante 40.

Le rayonnement lumineux LE est acheminé à l'extrémité non pénétrante de la branche 23A par une fibre optique gainée FOE. Dans le tube 25A, le rayonnement lumineux LE est d'abord guidé par une fibre optique 27A, et ensuite acheminé en espace libre jusqu'à la fenêtre 30A. Enfin, le rayonnement lumineux LE est guidé en espace libre depuis la fenêtre 30A jusqu'au morceau de viande VI.

Le rayonnement lumineux LE éclaire la viande VI à 90° par rapport à l'axe longitudinal de pénétration de la branche 23A.

Le diamètre du rayonnement lumineux d'émission LE est de l'ordre de quelques mm à travers la fenêtre 30A, qui est par exemple en quartz de 1 à 2 mm d'épaisseur.

Un miroir 32A est placé entre la sortie de la fibre 27A et la fenêtre 30A. Le miroir 32A est orienté à 45° par rapport à l'axe longitudinal de pénétration de la branche d'émission 23A pour réfléchir à 90° la lumière issue de la fibre optique 27A.

Une optique d'adaptation 34A, de type lentille par exemple, est placée entre la sortie de la fibre optique d'émission 27A et le miroir 30A pour adapter le rayonnement lumineux d'émission LE au volume de viande à observer.

La lumière transmise LT par le morceau de viande VI est reçue en espace libre par la fenêtre 30B, disposée perpendiculairement par rapport à l'axe de pénétration de la branche de réception 23B.

Le diamètre du rayonnement lumineux transmis LT est de l'ordre de quelques mm. La réception du rayonnement lumineux transmis LT s'effectue à travers une fenêtre 30B en quartz de 1 à 2 mm d'épaisseur.

Un miroir 32B est orienté à 45° par rapport à l'axe longitudinal de pénétration de la branche de réception 23B pour réfléchir la lumière transmise LT vers une fibre optique de réception 27B, disposée parallèlement à l'axe longitudinal de pénétration de la branche 23B.

Une optique d'adaptation 34B, de type lentille par exemple, est prévue entre le miroir 32B et l'entrée de la fibre optique 27B, pour adapter le rayonnement lumineux transmis à l'ouverture de la fibre optique 27B.

La lumière transmise LT est ensuite acheminée vers des moyens de mesure 50 par une fibre optique de réception gainée FOR.

De préférence, les moyens de mesure 50 comprennent un spectrophotomètre SPH, équipé d'un réseau (non représenté) qui disperse la lumière en différentes longueur d'ondes allant du visible jusqu'au proche infrarouge. La lumière diffractée par le réseau frappe une barrette de détection de type photodiodes capables chacune d'enregistrer l'intensité lumineuse transmise dans une gamme de longueurs d'ondes choisie.

Par exemple, la barrette de détection comprend des détecteurs en silicium pour les longueurs d'ondes inférieures à 1050 nm et en germanium pour les longueurs supérieures à 1050 nm.

Un bloc amplification (non représenté) est prévu après le bloc détection pour atteindre un niveau de signal situé entre 1 et 10 volts par exemple.

Par exemple, la source de lumière 40 est une lampe halogène au tungstène d'une puissance de 20 à 40 Watts. La particularité d'une telle lampe est de pouvoir émettre dans une large bande de fréquence un rayonnement lumineux allant par exemple du visible jusqu'au proche infrarouge.

La sonde 23 peut être utilisée manuellement.

Dans ce cas, elle peut être non solidaire de la source de lumière 40 et des moyens de mesure 50.

Avantageusement, la source de lumière et les moyens de mesure sont assemblés en un bloc commun 52, relié à un ordinateur 11.

Les fibres optiques FOE et FOR ont une longueur de l'ordre de 1 à 2m afin de minimiser les atténuations. Les diamètres des fibres optiques sont l'ordre de quelques mm.

L'émission de la lumière LE est avantageusement coupée par un disque en rotation (non représenté) situé sur le chemin optique d'émission, tournant par exemple à la fréquence de 20 à 60 Hertz, pour permettre des mesures du signal transmis en l'absence ou en présence de lumière émise.

Cette obturation cadencée permet de s'affranchir des variations de la lumière extérieure.

Il est à remarquer que le dispositif selon l'invention peut être réalisé sous la forme d'un boîtier comprenant une lampe 40 et des moyens de mesure 50 intégrés à la sonde 23 dans un seul boîtier susceptible d'être porté.

Les mesures spectrales peuvent aussi être obtenues en mode réflexion, avec application d'une sonde en surface ou en pénétration dans la viande dont la tendreté est à prédire.

Une sonde fonctionnant en réflexion est plus simple qu'une sonde fonctionnant en transmission.

Par exemple, en référence à la figure 5, la sonde 100 comprend généralement une seule branche 102 assurant le trajet aller et le trajet retour de la lumière incidente LI et de la lumière réfléchie LR.

La branche 102 comprend une seule fenêtre 104 disposée sur le côté interne de son extrémité pénétrante, perpendiculairement à l'axe de pénétration.

La branche comprend un tube 106, similaire à ceux décrits en référence à la figure 4.

Le rayonnement lumineux LI est émis par une source de lumière. Le rayonnement lumineux LI est acheminé à l'extrémité non pénétrante de la branche 102 par une fibre optique gainée FOER. Dans le tube 106, le rayonnement lumineux LI est d'abord guidé par une fibre optique 114, et ensuite acheminé en espace libre jusqu'à la fenêtre 104. Enfin, le rayonnement lumineux LI est guidé en espace libre depuis la fenêtre 104 jusqu'au morceau de viande VI.

Le rayonnement lumineux LI éclaire la viande VI à 90° par rapport à l'axe longitudinal de pénétration de la branche.

Le diamètre du rayonnement lumineux d'émission LI est de l'ordre de quelques mm à travers la fenêtre 104, qui est par exemple en quartz de 1 à 2 mm d'épaisseur.

Un miroir 110 est placé entre la sortie de la fibre 114 et la fenêtre 104. Le miroir 104 est orienté à 45° par rapport à l'axe longitudinal de pénétration de la branche 102 pour réfléchir à 90° la lumière issue de la fibre optique 114.

Une optique d'adaptation 112, de type lentille par exemple, est placée entre la sortie de la fibre optique 114 et le miroir 110 pour adapter le rayonnement lumineux d'émission LI au volume de viande à observer.

La lumière réfléchie LR par le morceau de viande VI est reçue en espace libre par la fenêtre 104 et traverse la branche dans le sens inverse de celui de la propagation de la lumière incidente LI. La lumière réfléchie LR est ensuite acheminée vers les moyens de mesure au moyen de la fibre optique d'émission/réception FOER.

Comme la sonde décrite en référence aux figures 3 et 4, la sonde 100 est reliée à une source de lumière et un spectrophotomètre qui peuvent être similaires à ceux décrits en référence aux figures 3 et 4. En pratique, la source et le spectrophotomètre sont réunis dans un bloc commun relié à la sonde par une seule fibre optique FOER assurant le transport de la lumière incidente ainsi que le transport de la lumière réfléchie.

La source lumineuse peut comprendre en amont un monochromateur dont le principe dispersif permet le balayage de la plage spectrale de 400 à 2500 nm.

La prédiction selon l'invention est objective, non destructive, et mise en place sur le site de transformation en milieu industriel sans faire appel à des dispositifs sophistiqués et coûteux. D'une manière générale, la fiabilité est meilleure lorsque de nombreuses données spectrales DSP et non spectrales DAC sont intégrées dans le modèle de prédiction. Toutefois, quelques paramètres (1 à 5 par exemple) de type DAC (qui peuvent varier d'un modèle de prédiction à l'autre selon le morceau de viande analysé, l'animal, et l'homogénéité de la population) en association avec des données spectrales DSP peuvent suffire pour atteindre une fiabilité satisfaisante.

**Revendications**

1. Procédé de prédiction de la qualité d'une viande en particulier de type bovin, sur le site de transformation, **caractérisé en ce qu'**il comprend les étapes suivantes :

   - a) recueillir, sur le site de transformation, des données relatives à des paramètres appartenant au groupe formé par la race, l'âge, et la catégorie de l'animal, ainsi qu'à des paramètres biologiques et/ou physico-chimiques de la carcasse de l'animal appartenant au groupe formé par le poids, la conformation, l'état d'engraissement, le pH et la couleur de la carcasse, ainsi que l'épaisseur du cuir,

   - b) obtenir au moins un spectre optique de la viande à des longueurs d'onde appartenant à une plage spectrale allant du visible jusqu'au proche infrarouge, et

- c) combiner les données obtenues lors des étapes a) et b), en vue de prédire la tendreté de la viande selon une loi de prédiction prédéterminée établie par rapport à une échelle de référence de tendreté prédéterminée.

2. Procédé selon la revendication 1, **caractérisé en ce que** l'étape b) est réalisée en mode réflexion et/ou en mode transmission.

3. Procédé selon la revendication 2, **caractérisé en ce que** l'étape b) comprend les étapes suivantes:

   - b1) prévoir une sonde (23) comprenant une branche d'émission (23A) et une branche de réception (23B), espacées l'une de l'autre d'une distance prédéterminée (26);

   - b2) insérer les branches d'émission et de réception (23A et 23B) dans un morceau de viande choisi (VI), à une profondeur choisie;

   - b3) éclairer le morceau de viande à l'aide de la branche d'émission (23A) ainsi insérée dans le morceau de viande, selon un rayonnement lumineux (LE) large bande en fréquence allant du visible jusqu'au proche infrarouge;

   - b4) recevoir la lumière transmise (LT) par le morceau de viande à l'aide de la branche réceptrice (23B) ainsi insérée dans le morceau de viande ; et

   - b5) enregistrer un spectre de transmission du morceau de viande allant du visible jusqu'au proche infrarouge.

4. Procédé selon la revendication 1, **caractérisé en ce que** la loi de prédiction est établie sur une série significative de différentes viandes pour chacune desquelles des données spectrales et non spectrales sont obtenues selon les étapes a) et b) et sont comparées à l'échelle de référence établie au moyen d'informations sensorielles et/ou de force de cisaillement et/ou de force de compression mesurées sur ladite série significative de différentes viandes.

5. Procédé selon la revendication 1, **caractérisé en ce que** la loi de prédiction est réalisée selon une méthode statistique multidimensionnelle du type méthode des moindres carrés partiels, en vue d'obtenir un modèle mathématique de prédiction destiné à être utilisé sur chaque morceau de viande dont la tendreté est à prédire.

6. Procédé selon la revendication 1, **caractérisé en ce que** les variables spectrales sont traitées selon une analyse du type composantes principales ou analogue.

7. Procédé selon la revendication 1, **caractérisé en ce que** l'étape b) est réalisée sur carcasses, quartiers, muscles ou steaks.

8. Dispositif de prédiction de la qualité d'une viande, en particulier de type bovin sur le site de transformation, **caractérisé en ce qu'**il comprend :

   - des moyens pour recueillir des données relatives à des paramètres appartenant au groupe formé par la race, l'âge, et la catégorie de l'animal, ainsi qu'à des paramètres biologiques et/ou physico-chimiques de la carcasse de l'animal appartenant au groupe formé par le poids, la conformation, l'état d'engraissement, le pH et la couleur de la carcasse, ainsi que l'épaisseur du cuir,

   - des moyens pour obtenir au moins un spectre optique de la viande à des longueurs d'ondes appartenant à une plage spectrale allant du visible jusqu'au proche infrarouge, et

   - des moyens de traitement pour combiner les données obtenues lors des étapes a) et b), en vue de prédire la tendreté de la viande, selon une loi de prédiction établie par rapport à une échelle de référence de tendreté prédéterminée.

9. Dispositif selon la revendication 8, **caractérisé en ce que** les moyens d'obtention de spectre comprennent:

   - une sonde (23) comprenant une branche d'émission (23A) et une branche de réception (23B), espacées l'une de l'autre d'une distance prédéterminée (26), et aptes à être insérées dans un morceau de viande choisi (VI), à une profondeur choisie;

   - une source de lumière (40) apte à délivrer un rayonnement lumineux large bande en fréquence allant du visible jusqu'au proche infrarouge, et optiquement connectée à la branche d'émission (23A) pour éclairer le morceau de viande à l'aide de ladite branche d'émission ainsi insérée dans le morceau de viande;

   - des moyens de mesure (50) aptes à enregistrer un spectre de transmission du morceau de viande allant du visible jusqu'au proche infrarouge et optiquement connectés à la branche réceptrice pour mesurer l'intensité de la lumière transmise par le morceau de viande et reçue par ladite branche réceptrice ainsi insérée dans le morceau de viande.

10. Dispositif selon la revendication 9, **caractérisé en ce que** la branche d'émission (23A) comprend une fenêtre (30A) disposée perpendiculairement à l'axe longitudinal de pénétration de la branche d'émission et au moins une fibre optique d'émission (FOE) reliant optiquement la source de lumière (40) à la fenêtre (30A) de la première branche (23A).

11. Dispositif selon la revendication 9, **caractérisé en ce que** la branche de réception (23B) comprend une fenêtre (30B) disposée perpendiculairement à l'axe longitudinal de pénétration de la branche de réception, en regard de la fenêtre (30A) de la branche d'émission, et au moins une fibre optique de réception (FOR) reliant optiquement la fenêtre (30B) de la seconde branche (23B) aux moyens de mesure (50).

12. Dispositif selon la revendication 9, **caractérisé en ce que** la distance (26) entre les deux branches (23A et 23B) est réglable afin de réaliser des mesures optiques dans un volume de viande (VI) de l'ordre de quelques cm$^3$.

13. Dispositif selon la revendication 8, **caractérisé en ce que** les moyens d'obtention de spectre comprennent:

- une sonde (100) comprenant une branche d'émission/réception (102) apte à être insérée dans un morceau de viande choisi (VI), à une profondeur choisie;

- une source de lumière apte à délivrer un rayonnement lumineux (LI) large bande en fréquence allant du visible jusqu'au proche infrarouge, et optiquement connectée à la branche d'émission/réception (102) pour éclairer le morceau de viande à l'aide de ladite branche d'émission/réception ainsi insérée dans le morceau de viande; et

- des moyens de mesure aptes à enregistrer un spectre de réflexion du morceau de viande allant du visible jusqu'au proche infrarouge et optiquement connectés à la branche d'émission/réception (102) pour mesurer l'intensité de la lumière réfléchie (LR) par le morceau de viande et reçue par ladite branche d'émission/réception ainsi insérée dans le morceau de viande.

**Patentansprüche**

1. Verfahren zum Vorhersagen der Qualität von Fleisch, insbesondere vom Typ Rindfleisch, am Ort der Verarbeitung, **dadurch gekennzeichnet, daß** es die folgenden Schritte aufweist:

- a) am Ort der Verarbeitung Erfassen von Daten bezüglich der Kenngrößen, die zur Gruppe gehören, die durch die Rasse, das Alter und die Gattung des Tieres gebildet wird, sowie bezüglich der biologischen und/oder physikalisch-chemischen Kenngrößen des Schlachttierkörpers, die zur Gruppe gehören, die durch das Gewicht, die Gestalt, den Fettzustand, den pH-Wert und die Farbe des Schlachttierkörpers, sowie die Dicke der Haut gebildet wird,

- b) Erhalten von mindestens einem optischen Spektrum des Fleisches bei Wellenlängen, die zu einem Spektralbereich gehören, der vom sichtbaren bis zum nahen Infrarot reicht, und

- c) Kombinieren der während der Schritte a) und b) erhaltenen Daten im Hinblick auf das Vorhersagen der Zartheit des Fleisches gemäß einer festgelegten Vorhersageregel, die bezüglich einer Referenzskala von festgelegter Zartheit aufgestellt wurde.

2. Verfahren nach Anspruch 1, **dadurch gekennzeichnet, daß** der Schritt b) im Reflexions- und/oder Transmissionsmodus ausgeführt wird.

3. Verfahren nach Anspruch 2, **dadurch gekennzeichnet, daß** der Schritt b) die folgenden Schritte aufweist:

- b1) Vorsehen einer Sonde (23), die einen Sendearm (23A) und einen Empfangsarm (23B) aufweist, die voneinander in einem vorbestimmten Abstand (26) beabstandet sind;

- b2) Einführen der Sende- und Empfangsarme (23A und 23B) in ein ausgewähltes Stück Fleisch (VI) in einer ausgewählten Tiefe;

- b3) Beleuchten des Stück Fleisches mittels des auf diese Weise in das Stück Fleisch eingeführten Sendearms (23A), gemäß einer breitbandigen Lichtbestrahlung (LE), deren Frequenz vom sichtbaren bis zum nahen Infrarot reicht;

- b4) Empfangen des Durchlichts (LE) durch das Stück Fleisch mittels des auf diese Weise in das Stück Fleisch eingeführten Empfangsarms (23B); und

- b5) Aufnehmen eines Transmissionsspektrums des Stücks Fleisch, das vom sichtbaren bis zum nahen Infrarot reicht.

4. Verfahren nach Anspruch 1, **dadurch gekennzeichnet, daß** die Vorhersageregel für eine bedeutsame Reihe von unterschiedlichen Fleischsorten aufgestellt wird, wobei für jede Fleischsorte Spektralwerte und Nichtspektralwerte gemäß der Schritte a) und b) erfaßt werden und mit der Referenzskala verglichen werden, die mit Hilfe der Meßinformationen und/oder der Scherkraft und/oder der Preßkraft, die für besagte bedeutsame Serie von unterschiedlichen Fleischsorten gemessen wurden, gebildet wurde.

5. Verfahren nach Anspruch 1, **dadurch gekennzeichnet, daß** die Vorhersageregel gemäß einer statistischen, mehrdimensionalen Methode vom Typ Methode der kleinsten Quadrate (Partial Least Squares) ausgeführt wird, um ein mathematisches Vorhersagemodell zu erhalten, das dazu bestimmt ist, für jedes Stück Fleisch verwendet zu werden, dessen Zartheit vorherzusagen ist.

6. Verfahren nach Anspruch 1, **dadurch gekennzeichnet, daß** die Spektralvariablen gemäß einer Analyse vom Typ Analyse der Hauptkomponenten oder dergleichen verarbeitet werden.

7. Verfahren nach Anspruch 1, **dadurch gekennzeichnet, daß** der Schritt b) bei Schlachttierkörpern, Vierteln, Muskeln oder Steaks ausgeführt wird.

8. Vorrichtung zum Vorhersagen der Qualität von Fleisch, insbesondere vom Typ Rindfleisch, am Ort der Verarbeitung, **dadurch gekennzeichnet, daß** sie aufweist:

   - Mittel zum Erfassen von Daten bezüglich der Kenngrößen, die zur Gruppe gehören, die durch die Rasse, das Alter und die Gattung des Tieres gebildet wird, sowie bezüglich der biologischen und/oder physikalisch-chemischen Kenngrößen des Schlachttierkörpers, die zur Gruppe gehören, die durch das Gewicht, die Gestalt, den Fettzustand, den pH-Wert und die Farbe des Schlachttierkörpers, sowie die Dicke der Haut gebildet wird,

   - Mittel zum Erhalten mindestens eines optischen Spektrums des Fleisches bei Wellenlängen, die zu einem Spektralbereich gehören, der vom sichtbaren bis zum nahen Infrarot reicht, und

   - Verarbeitungsmittel zum Kombinieren der während der Schritte a) und b) erhaltenen Werte, um die Zartheit des Fleisches gemäß einer Vorhersageregel vorherzusagen, die in Bezug auf eine Referenzskala von vorbestimmter Zartheit festgelegt wurde.

9. Vorrichtung nach Anspruch 8, **dadurch gekennzeichnet, daß** die Mittel zum Erhalten des Spektrums aufweisen:

   - eine Sonde (23), die einen Sendearm (23A) und einen Empfangsarm (23B) enthält, die voneinander in einem vorbestimmten Abstand (26) beabstandet sind, und die geeignet sind, in ein ausgewähltes Stück Fleisch (VI) in einer ausgewählten Tiefe eingeführt zu werden;

   - eine Lichtquelle (40), geeignet, um eine breitbandige Lichtstrahlung einer Frequenz, die vom sichtbaren bis zum nahen Infrarot reicht, zu liefern, und die optisch mit dem Sendearm (23A) verbunden ist, um das Stück Fleisch mittels des auf diese Weise in das Stück Fleisch eingeführten Sendearms zu beleuchten;

   - Meßmittel (50), die geeignet sind, ein Transmissionsspektrum des Stücks Fleischs aufzunehmen, das vom sichtbaren bis zum nahen Infrarot reicht, und die optisch mit dem Empfangsarm verbunden sind, um die Intensität des durch das Stück Fleisch transmittierten und durch besagten, auf diese Weise in das Stück Fleisch eingeführten Empfangsarms empfangenen Lichts zu messen.

10. Vorrichtung nach Anspruch 9, **dadurch gekennzeichnet, daß** der Sendearm (23A) ein Fenster (30A), das senkrecht zur Längsachse der Penetration des Sendearms angeordnet ist, und mindestens eine Sendeglasfaser (FOE) enthält, die optisch die Lichtquelle (40) mit dem Fenster (30A) des ersten Arms (23A) verbindet.

11. Vorrichtung gemäß Anspruch 9, **dadurch gekennzeichnet, daß** der Empfangsarm (23B) ein Fenster (30B), das senkrecht zur Längsachse der Penetration des Empfangsarms, in Richtung des Fensters (30A) des Sendearms angeordnet ist, und mindestens eine Empfangsglasfaser (FOR) aufweist, die optisch das Fenster (30B) des zweiten Arms (23B) mit den Meßmitteln (50) verbindet.

12. Vorrichtung gemäß Anspruch 9, **dadurch gekennzeichnet, daß** der Abstand (26) zwischen den beiden Armen (23A und 23B) einstellbar ist, um optische Messungen in einem Fleischvolumen (VI) der Größenordnung einiger cm$^3$ durchzuführen.

**13.** Vorrichtung gemäß Anspruch 8, **dadurch gekennzeichnet, daß** die Mittel zum Erhalten des Spektrums aufweisen:

- eine Sonde (100), die eine Sende-/Empfangsarm (102) aufweist, geeignet, um in ein ausgewähltes Stück Fleisch (VI) in einer ausgewählten Tiefe eingeführt zu werden;

- eine Lichtquelle, die geeignet ist, eine breitbandige Lichtstrahlung (LI) einer Frequenz, die vom sichtbaren bis zum nahen Infrarot reicht, zu liefern, und die optisch mit dem Sende-/ Empfangsarm (102) verbunden ist, um das Stück Fleisch mittels des besagten, auf diese Weise in das Stück Fleisch eingeführten Sende/Empfangsarms zu beleuchten; und

- Meßmittel, die geeignet sind, ein Reflexionsspektrum des Stück Fleisches aufzunehmen, das vom sichtbaren bis zum nahen Infrarot reicht, und die optisch mit dem Sende-/Empfangsarm (102) verbunden sind, um die Intensität des durch das Stück Fleisch reflektierten (LR) und durch besagten, auf diese Weise in das Stück Fleisch eingeführten Sende-/Empfangsarm empfangenen Lichts zu messen.

**Claims**

**1.** Process for predicting the quality of a meat, particularly of the bovine type, at the conversion site, **characterised in that** it comprises the following steps:

a) collecting, at the conversion site, data relating to parameters pertaining to the group consisting of the breed, age and category of the animal, and also biological and/or physico- chemical parameters of the carcase of the animal pertaining to the group consisting of the weight, build, state of fattening, pH and colour of the carcase, and the thickness of the hide, b) obtaining at least one optical spectrum of the meat at wavelengths belonging to a spectral range going from visible to near infrared, and c) combining the data obtained in steps a) and b) in order to predict the tenderness of the meat according to a predetermined law of prediction drawn up in relation to a predetermined tenderness reference scale.

**2.** Process according to claim 1, **characterised in that** step b) is carried out in reflective mode and/or in transmissive mode.

**3.** Process according to claim 2, **characterised in**

**that** step b) comprises the following steps:

b1) providing a probe (23) comprising a transmitting branch (23A) and a receiving branch (23B) spaced at a predetermined distance (26) from one another;

b2) inserting the transmitting and receiving branches (23A and 23B) in a selected piece of meat (VI) to a selected depth;

b3) illuminating the piece of meat by means of the transmitting branch (23A) thus inserted in the piece of meat, according to wideband light radiation (LE) ranging in frequency from visible to near infrared;

b4) receiving the light (LT) transmitted by the piece of meat using the receiving branch (23B) thus inserted in the piece of meat; and

b5) recording a transmission spectrum of the piece of meat ranging from visible to near infrared.

**4.** Process according to claim 1, **characterised in that** the law of prediction is drawn up over a significant series of different meats, for each of which spectral and non-spectral data are obtained according to steps a) and b) and are compared with the reference scale drawn up using sensory information and/or shearing force data and/or compressive force data measured on said significant series of different meats.

**5.** Process according to claim 1, **characterised in that** the law of prediction is drawn up using a multi- dimensional statistical method of the partial least squares method type, with a view to obtaining a mathematical prediction model intended to be used on each piece of meat the tenderness of which is to be predicted.

**6.** Process according to claim 1, **characterised in that** the spectral variables are processed by principal component analysis or the like.

**7.** Process according to claim 1, **characterised in that** step b) is carried out on carcases, quarters, muscles or steaks.

**8.** Device for predicting the quality of a meat, particularly of the bovine type, at the conversion site, **characterised in that** it comprises:

- means for collecting data relating to parameters pertaining to the group consisting of the breed, age and category of the animal, and also biological and/or physico-chemical parameters of the carcase of the animal pertaining to the group consisting of the weight, build, state of fattening, pH and colour of the carcase, and the thickness of the hide,

- means for obtaining at least one optical spectrum of the meat at wavelengths belonging to a spectral range going from visible to near infrared, and

- means for combining the data obtained in steps a) and b) in order to predict the tenderness of the meat according to a law of prediction drawn up in relation to a predetermined tenderness reference scale.

9. Device according to claim 8, **characterised in that** the means for obtaining spectra comprise:

- a probe (23) comprising a transmitting branch (23A) and a receiving branch (23B) spaced at a predetermined distance (26) from one another and adapted to be inserted in a selected piece of meat (VI) to a selected depth;

- a light source (40) adapted to deliver wide band light radiation ranging in frequency from visible to near infrared, and optically connected to the transmitting branch (23A) in order to illuminate the piece of meat using said transmitting branch thus inserted in the piece of meat;

- measuring means (50) adapted to record a transmission spectrum of the piece of meat ranging from visible to near infrared and optically connected to the receiving branch in order to measure the intensity of the light transmitted by the piece of meat and received by said receiving branch thus inserted in the piece of meat.

10. Device according to claim 9, **characterised in that** the transmitting branch (23A) has a window (30A) disposed perpendicularly with respect to the longitudinal axis of penetration of the transmitting branch and at least one transmitting optical fibre (FOE) optically connecting the light source (40) to the window (30A) of the first branch (23A).

11. Device according to claim 9, **characterised in that** the receiving branch (23B) has a window (30B) disposed perpendicularly with respect to the longitudinal axis of penetration of the receiving branch, opposite the window (30A) of the transmitting branch, and at least one receiving optical fibre (FOR) optically connecting the window (30B) of the second branch (23B) to the measuring means (50).

12. Device according to claim 9, **characterised in that** the distance (26) between the two branches (23A and 23B) is adjustable in order to carry out optical measurements in a volume of meat (VI) of the order of a few cm$^3$.

13. Device according to claim 8, **characterised in that** the means for obtaining spectra comprise:

- a probe (100) comprising a transmitting/receiving branch (102) adapted to be inserted in a selected piece of meat (VI) to a selected depth;

- a light source adapted to deliver wide band light radiation (LI) ranging in frequency from visible to near infrared, and optically connected to the transmitting/receiving branch (102) in order to illuminate the piece of meat using said transmitting/receiving branch thus inserted in the piece of meat; and

- measuring means adapted to record a reflection spectrum of the piece of meat ranging from visible to near infrared and optically connected to the transmitting/receiving branch (102) in order to measure the intensity of the light reflected (LR) by the piece of meat and received by said transmitting/receiving branch thus inserted in the piece of meat.

FIG.1

SPECTRES OBTENUS EN REFLEXION SUR FAUX FILETS DE VACHES DURS ET TENDRES

FIG.2

FIG.3

FIG.4

FIG.5